# EUROPÄISCHE PATENTANMELDUNG

(11) **EP 0 947 220 A2**
(43) Veröffentlichungstag der Anmeldung: **06.10.1999**
(21) Anmeldenummer: 99103859.7
(22) Anmeldetag: 28.02.1999
(51) Int. Cl.: A61N 5/06

(54) **Bestrahlungsliege**

(30) Priorität: 09.03.1998 DE 19809795
(71) Anmelder: Kopper, Iris, 44227 Dortmund (DE)
(72) Erfinder: Pürschel, Dieter, 44227 Dortmund (DE)
(74) Vertreter: Grosse, Dietrich, Dipl.-Ing.

(57) **Zusammenfassung**

Eine Bestrahlungsliege, die aus einem, auf einen Sockel (FS) aufsetzbaren Liegenkörper (LK) in der Form eines rinnenförmigen Hohlzylinderabschnitts besteht und aus einem, die Bestrahlungseinrichtungen aufnehmenden, ebenfalls hohlzylinderabschnittsförmigen Himmel-Körper (HK), der an eine Zylinderlängskante des Liegenkörpers (LK) schwenkbar angelenkt ist. Der Liegenkörper (LK) und/oder der Himmelkörper (HK) bestehen aus jeweils einem Paar sichelförmiger Spantenelemente 5;4 und aus einem Paar, diese mit Abstand verbindender Profilleisten (3;2), die die parallelen Zylinderlängskanten bilden. Auf die Spantenelemente (5;4) sind Abdeckplatten (6,7) auflegbar, die die Aussen- bzw. Innenwände bilden und die Bestrahlungseinrichtungen aufnehmen. Diese Abdeckplatten (6,7) sind lösbar mit den Profilleisten (3;2) und den Spantenelementen (5;4) verbindbar

## Beschreibung

Die Erfindung betrifft eine Bestrahlungsliege, bestehend aus einem, auf einen Sockel aufsetzbaren Liegen-Körper in der Form eines rinnenförmigen Hohlzylinderabschnitts und einem, an eine Zylinderlängskante des Liegen-Körpers schwenkbar angelenkten, die Bestrahlungseinrichtungen aufnehmenden, ebenfalls hohlzylinderabschnittsförmigen Himmel-Körper.

Liegen dieser Art sind in vielen Ausbildungsformen bekanntgeworden. Von diesen sind die, für den gewerblichen Gebrauch z.B. in Bädern, Sonnenstudios u. dergl. bestimmten Ausführungen verhältnismäßig schwer gebaut, mit der Folge, daß nicht nur die Herstellung des Liegengestells und dessen Zusatzeinrichtungen, sondern auch deren Transport zum Aufstellungsort und dort die Zusammenstellung und Montage einen erheblichen Arbeitsaufwand und dazu auch erfahrenes Personal erfordern.

Der Erfindung liegt die Aufgabe zugrunde, eine Bestrahlungsliege zu schaffen, deren Herstellung und deren Transport und Aufbau am Aufstellungsort erheblich erleichtert sind und der Zusammenbau und Einstellung keine Fachkräfte erfordert.

Diese Aufgabe wird dadurch gelöst, daß der Liegen-Körper und/oder der Himmel-Körper aus jeweils einem Paar, deren parallele Zylinderlängskanten bildende Profilleisten und, diese mit Abstand verbindenden sichelförmigen Spanten-Elementen bestehen, auf die, die Außen- bzw. die Innenwände der Körper bildende Abdeckplatten auflegbar sind, die lösbar mit den Profilleisten und/oder den Spanten-Elementen verbindbar sind.

Die Abdeckplatten können dabei erfindungsgemäß aus flexiblen Werkstoffen, z.B. lichtundurchlässigen oder lichtdurchlässigen, ggfs. gefärbten Kunststoffen oder Metallen, wie Aluminium bestehen. Wie die Erfindung weiter vorsieht, können auf diese Abdeckplatten flexible Dekorplatten auflegbar sein. Die Längskanten der Abdeckplatten und ggfs. auch die der Dekorplatten können in Längsnuten der Profilleisten einsteckbar sein. Die Längskanten der Dekorplatten können, wie die Erfindung weiter vorsieht, von, die Profilleisten einfassenden Profilrinnen gehalten werden, die dabei auch als Berührungsschutzleisten ausgebildet sein können.

Auf die Spanten-Elemente, die den Himmel-Körper beiderseits begrenzen, können erfindungsgemäß mit Durchtrittsausnehmungen versehene Blenden, Filterscheiben und/oder Dekorelemente aufgesetzt werden.

In die rückwärtige Profilleiste des Himmel-Körpers kann, wie die Erfindung weiter vorsieht, eine Drehfeder mit Drehfedergehäuse eingelegt werden, deren beide Achsenden in, nach oben offene Lagergabeln einlegbar sind, die mit den, den Liegen-Körper beidseitig begrenzenden Spanten-Elementen verbunden sind. Dabei kann die Drehfeder an einem oder beiden Stirnenden eine, auf eine, die Stirnenden des Drehfedergehäuses begrenzende Flanschscheibe auflegbare Stellscheibe aufweisen, die mit einer Mehrzahl von Stellbohrungen für das Einbringen eines, die Stellscheibe gegen die Flanschscheibe des Drehfedergehäuses festlegenden Riegelbolzens versehen ist. Weiter können nach einem Vorschlag der Erfindung die Stellscheiben eine Mehrzahl von Gewindebohrungen für das Einbringen von Gewindebolzen aufweisen, deren Stirnenden die Flanschscheibe bremsbeaufschlagen.

Die Erfindung wird anhand des, in der Zeichnung dargestellten Ausführungsbeispiels näher erläutert. In der Zeichnung zeigen:
- Fig. 1: Die Einzelheiten der Bestrahlungsliege in perspektivischer Darstellung,
- Fig. 2, 3, 4 und 5: die Bestrahlungsliege in verschiedenen Zusammenbaupositionen in schematischer Darstellung,
- Fig. 6, 7 und 8: Einzelheiten des Liegen-Körpers in schematischer Darstellung und
- Fig. 9, 10 und 11: weitere Einzelheiten der Bestrahlungsliege ebenfalls in schematischer Darstellung.

Liegen-Körper LK und Himmel-Körper HK sind aus je einem Profilleistenpaar 2 bzw. 3 und diese mit ihren sichelförmigen Enden verbundenen Spanten-Elementen 4 bzw. 5 zusammengesetzt. Auf die Spanten-Elemente 4 ist von oben eine Abdeckplatte 6 aufgelegt. Die, zwischen den Spanten-Elementen 4 angeordneten, nicht dargestellten Bestrahlungsleuchten werden durch eine lichtdurchlässige Kunststoffplatte 7 abgedeckt. Auf die Abdeckplatte 6 ist eine auswechselbare Dekorplatte 8 auflegbar. In die rückwärtige Profilleiste 2 ist eine, nicht dargestellte Drehfeder mit Drehfedergehäuse eingesetzt, deren Achsenden 21 aus den, die Seiten des Himmel-Körpers HK begrenzenden Spanten-Elementen 4 herauskragen. Auf die Spanten-Elemente 5 des Liegen-Körpers LK ist eine Abdeckplatte 17, von unten her aufgelegt, auf die ebenfalls eine Dekorplatte 9 auflegbar ist. Die, nicht dargestellten Bestrahlungsleuchten werden ebenso wie die des Himmel-Körpers HK durch eine lichtdurchlässige Kunststoffplatte 10 abgedeckt. Auf die die Seiten des Liegen-Körpers LK begrenzenden Spanten-Elemente 5 sind, nach oben offene Lagergabeln 22 aufgesetzt, in die, wie später noch näher erläutert, die Achsenden 21 der, in die Profilleiste 2 des Himmel-Körpers HK eingesetzten Drehfeder eingelegt werden.

Der Liegen-Körper LK wird auf einen Fuß-Sockel FS aufgesetzt und, wie später noch näher erläutert, mit diesem schraubverbunden. Er nimmt, nicht dargestellte Steuer- und andere Betriebsaggregate für die Bestrahlungsliege auf.

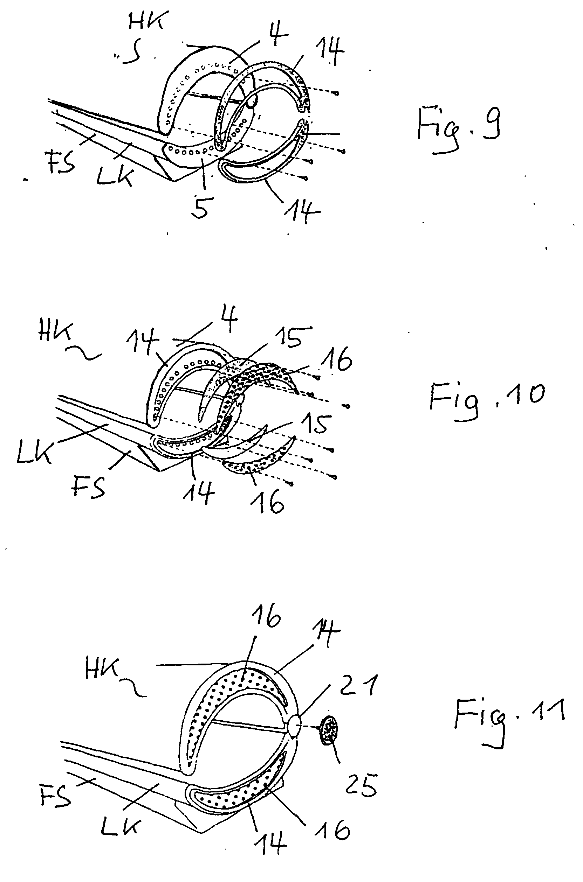

Wie aus Fig. 2 zu ersehen wird die, auf die Abdeckplatte 17, hier des Liegen-Körpers LK aufgelegte Dekorplatte 9 von Profilrinnen 18 gehalten, die die mit den Enden der Spanten-Elemente 5 verbundenen Profilleisten einfassen. Diese Profilrinnen 18 dienen dabei, wie ersichtlich, gleichzeitig als Berührungsschutzleisten.

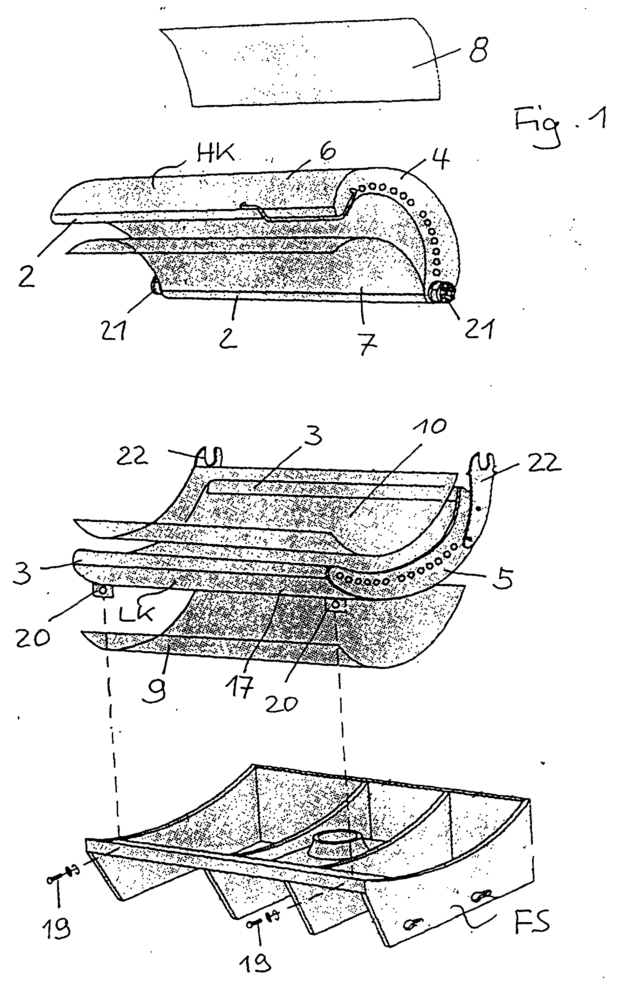

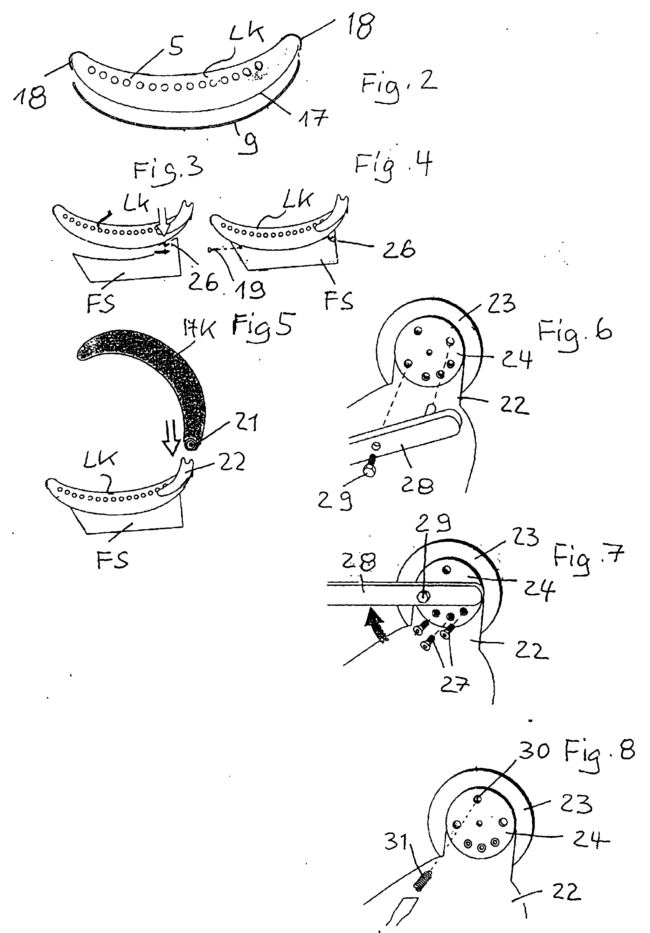

Fig. 3 und 4 zeigen, wie der Liegen-Körper LK in einfacher Weise auf den Fuß-Sockel FS aufgesetzt, mit rückwärtigen Fanghaken 26 festgerastet und mit diesem durch Schrauben 19 und Ösen 20 verschraubt wird (vergl. auch Fig. 1).

Der Himmel-Körper HK wird, wie aus Fig. 5 hervorgeht, mit den beiden Achsenden 21 der, in der rückwärtigen Profilleiste 2 angeordneten Drehfeder in die Lagergabeln 22 des Liegen-Körpers LK eingelegt und kann, um diese Achsenden 21 geschwenkt, aus der dargestellten Öffnungsposition in die Schließposition gebracht werden.

Wie aus den Fig. 6 bis 8 hervorgeht, sitzt auf dem Stirnende der nicht dargestellten Drehfeder eine Flanschscheibe 23, die mit Hilfe eines Spannhebels 28 mit Feststellschraube 29 gegen eine Stellscheibe 24 drehverstell- und festlegbar ist. Diese Stellscheibe 24 kann mit Hilfe von Schrauben 27 mit der Lagergabel 22 verbunden werden. In Gewindebohrungen 30 der Stellscheibe 24 sind Gewindebolzen 31 einschraubbar, deren Stirnenden die Flanschscheibe 23 bremsbeaufschlagen.

Aus den Fig. 9 - 11 ist zu ersehen, daß auf die äußeren seitlichen Begrenzungs-Spanten-Elemente 4 bzw. 5 des Himmel-Körpers HK und des Liegen-Körpers LK mit Durchtrittsöffnungen versehene Seitenblenden 14 aufsetzbar sind, in die Luftfilter 15 mit Filtergittern 16 eingelegt sind. Die Achsenden 21 der Drehfederanordnung lassen sich, wie Fig. 11 zeigt, mit Hilfe eines aufsteckbaren Dekorknopfes abdecken.

### Bezugszeichenverzeichnis

- 1:
- 2: Profilleiste
- 3: Profilleiste
- 4: Spanten-Element
- 5: Spanten-Element
- 6: Abdeckplatte
- 7: (lichtdurchlässige) Kunststoffplatte
- 8: Dekorplatte
- 9: Dekorplatte
- 14: Seitenblenden
- 15: Luftfilter
- 16: Filtergitter
- 17: Abdeckplatte
- 18: Profilrinne
- 19: Schrauben
- 20: Ösen
- 21: Achsenden
- 22: Lagergabel
- 23: Flanschscheibe
- 24: Stellscheibe
- 25: Dekorknopf
- 26: Fanghaken
- 27: Schrauben
- 28: Spannhebel
- 29: Feststellschraube
- 30: Gewindebohrung
- 31: Gewindebolzen
- LK: Liegen-Körper
- HK: Himmel-Körper
- FS: Fuß-Sockel

## Patentansprüche

1. Bestrahlungsliege, bestehend aus einem, auf einen Sockel aufsetzbaren Liegen-Körper in der Form eines rinnenförmigen Hohlzylinderabschnitts und einem, an eine Zylinderlängskante des Liegen-Körpers schwenkbar angelenkten, die Bestrahlungseinrichtungen aufnehmenden, ebenfalls hohlzylinderabschnittsförmigen Himmel-Körper,
**dadurch gekennzeichnet,**
daß der Liegen-Körper (LK) und/oder der Himmel-Körper (HK) aus jeweils einem Paar, die parallelen zylinderlängskanten bildenden Profilleisten (3 bzw. 2) und diese, mit Abstand verbindenden, sichelförmigen Spanten-Elementen (5 bzw. 4) bestehen, auf die, die Außen- bzw. die Innenwände der Körper bildende, sowie die Bestrahlungslichtquellen abdeckende Abdeckplatten (6, 7, 10, 17) auflegbar sind, die lösbar mit den Profilleisten (3 bzw. 2) und/oder den Spanten-Elementen (5 bzw. 4) verbindbar sind.

2. Bestrahlungsliege nach Anspruch 1,
**dadurch gekennzeichnet,**
daß die Abdeckplatten (6, 7, 10, 17) aus lichtundurchlässigen bzw. lichtdurchlässigen, ggfs. gefärbten Kunststoffen oder aus Metallen, wie Aluminium bestehen.

3. Bestrahlungsliege nach den Ansprüchen 1/oder 2,
**gekennzeichnet durch**
auf die Abdeckplatten (6, 17) auflegbare, ebenfalls flexible Dekorplatten (8, 9).

4. Bestrahlungsliege nach einem oder mehreren
der Ansprüche 1 bis 3,
**dadurch gekennzeichnet,**
daß die Längskanten der Abdeckplatten (6, 17 bzw. 7, 17) bzw. der Dekorplatten (8, 9) in Längsnuten der Profilleisten (2, 3) einsteckbar sind.

5. Bestrahlungsliege nach einem oder mehreren
der Ansprüche 1 bis 4,
**dadurch gekennzeichnet,**
daß die Längskanten der Dekorplatten (8, 9) von, die Profilleisten (2, 3) einfassenden Profilrinnen (18) gehalten werden.

6. Bestrahlungsliege nach Anspruch 5,
**dadurch gekennzeichnet,**
daß die Profilrinnen (18) als Berührungssschutzleisten ausgebildet sind.

7. Bestrahlungsliege nach einem oder mehreren
der Ansprüche 1 bis 6,
**gekennzeichnet durch**
auf die, den Himmel-Körper (HK) beidseitig begrenzenden Spanten-Elemente (4) aufsetzbare, ggfs. mit Durchtrittsausnehmungen versehene Blenden (14), Filterscheiben (15), Filtergitter (16) und/oder Dekorelemente.

8. Bestrahlungsliege nach einem oder mehreren
der Ansprüche 1 bis 7,
**gekennzeichnet durch**
eine in die rückwärtige Profilleiste (2) des Himmel-Körpers (HK) eingelegte Drehfeder mit Drehfedergehäuse, deren beide Achsenden (21) in, nach oben offene Lagergabeln (22), die mit den, den Liegen-Körper (LK) beidseitig begrenzenden Spanten-Elementen (5) verbunden sind.

9. Bestrahlungsliege nach Anspruch 8,
**dadurch gekennzeichnet,**
daß die Drehfeder an einem oder beiden Stirnenden eine, auf eine, die Stirnenden des Drehfedergehäuses begrenzende Flanschscheibe (23) auflegbare Stellscheibe (24) aufweist, die mit einer Mehrzahl von Stellbohrungen für das Einbringen eines, die Stellscheibe (24) gegen die Flanschscheibe (23) festlegenden Riegelbolzens (29 versehen ist.

10. Bestrahlungsliege nach den Ansprüchen 8 und/oder 9,
**dadurch gekennzeichnet,**
daß die Stellscheibe (24) Gewindebohrungen (30) für das Einbringen von Gewindebolzen (31) aufweist, deren Stirnenden die Flanschscheibe (23) bremsbeaufschlagen.
